(19) Europäisches Patentamt
European Patent Office
Office européen des brevets

(11) Veröffentlichungsnummer: **0 033 927**
**B1**

(12) # EUROPÄISCHE PATENTSCHRIFT

(45) Veröffentlichungstag der Patentschrift: **17.08.83**

(51) Int. Cl.³: **C 08 G 18/79** // C07D229/00

(21) Anmeldenummer: **81100714.5**

(22) Anmeldetag: **02.02.81**

(54) **Verfahren zur kontinuierlichen Herstellung von Uretdiongruppen aufweisenden Polyisocyanaten und ihre Verwendung als Aufbaukomponente bei der Herstellung von Polyurethankunststoffen.**

(30) Priorität: **12.02.80 DE 3005106**

(43) Veröffentlichungstag der Anmeldung: **19.08.81 Patentblatt 81/33**

(45) Bekanntmachung des Hinweises auf die Patenterteilung: **17.08.83 Patentblatt 83/33**

(84) Benannte Vertragsstaaten: **AT BE CH DE FR GB IT LI**

(56) Entgegenhaltungen:
**DE-A-2 044 838**
**DE-A-2 312 391**
**DE-A-2 420 475**
**DE-A-2 452 390**

(73) Patentinhaber: **BAYER AG, Zentralbereich Patente, Marken und Lizenzen, D-5090 Leverkusen 1, Bayerwerk (DE)**

(72) Erfinder: **Reischl, Artur, Dr., H.T.-von-Böttinger-Strasse 19, D-5090 Leverkusen 1 (DE)**
Erfinder: **Quiring, Bernd, Dr., Albrecht-Haushofer-Strasse 2, D-5090 Leverkusen 1 (DE)**
Erfinder: **Rathjen, Claus, Dl., Theodor-Heuss-Ring 78, D-5090 Leverkusen 1 (DE)**

**EP 0 033 927 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur kontinuierlichen Herstellung von Uretdiongruppen aufweisenden Polyisocyanaten und ihre Verwendung als Aufbaukomponente bei der Herstellung von Polyurethankunststoffen

Die vorliegende Erfindung betrifft ein Verfahren zur kontinuierlichen Herstellung von Uretdiongruppen aufweisenden Isocyanaten durch Dimerisierung von Polyisocyanaten in Gegenwart von eine oder mehrere Hydroxylgruppen aufweisenden Verbindungen und unter Mitverwendung von an sich bekannten Dimerisierungskatalysatoren, sowie die Verwendung der Verfahrensprodukte zur Herstellung von Polyurethankunststoffen.

Die Herstellung von polyfunktionellen Uretdionisocyanaten in Gegenwart von Dimerisierungskatalysatoren ist bekannt (vgl. Kunststoff-Handbuch, Band VII, Polyurethane, herausgegeben von Vieweg-Höchtlen, Carl-Hanser-Verlag, München, 1966, Seite 16).

Nach einer besonderen, in DE-OS 2 452 390 bzw. US-PS 3 993 641 beschriebenen Ausführungsform wird die Dimerisierungsreaktion in einem kontinuierlich arbeitenden kühlbaren Schneckenreaktor durchgeführt. Bei diesem Verfahren fällt das uretdiongruppenhaltige Polyisocyanat, z. B. dimerisiertes 2,4-Toluylendiisocyanat, in Form eines Pulvers an, das erst durch einen zusätzlichen Mahlschritt die für viele Arten der Verarbeitung notwendige Feinteiligkeit erhält. Bei dieser Art der Herstellung tritt ferner starker Verschleiß in der Schneckenmaschine durch Abrieb auf, der sich z. B. durch laute Quietschgeräusche im Verfahrensteil bemerkbar macht.

Schwierig ist in der beschriebenen Ausführungsform die Abführung der beträchtlichen Reaktionswärme durch das wenig leitfähige Produktpulver. Zur Erzielung eines reinen Dimerisats dürfen bestimmte, verhältnismäßig niedrig liegende Temperaturen nicht überschritten werden. Es ist naheliegend, daß in großen Maschinen deshalb nur unwirtschaftliche Durchsätze erreicht werden. Die in der US-Patentschrift 3 993 641 ebenfalls vorgeschlagene Mitverwendung von inerten Plastifizierungsmitteln führt schließlich zu externe Weichmacher enthaltenden Kunststoffen mit Nachteilen wie beispielsweise Ausschwitzen, hohem Gehalt an extrahierbaren Stoffen und allmähliche Versprödung der Endprodukte.

Es wurde nunmehr überraschend ein Verfahren gefunden, das es ermöglicht, Uretdiongruppen aufweisende Polyisocyanate in großen Maschinendurchsätzen in einem Arbeitsgang mit gegenüber Isocyanat reaktionsfähigen Verbindungen als Dispersionsmittel in sehr feinteiliger Form herzustellen, die die obengenannten Nachteile bei den Endprodukten nicht aufweisen.

Gegenstand der vorliegenden Erfindung ist ein Verfahren zur kontinuierlichen Herstellung von Uretdiongruppen aufweisenden Polyisocyanaten durch Dimerisierung von uretdiongruppenfreien aromatischen Polyisocyanaten in Gegenwart von die Dimerisierung von Isocyanaten beschleunigenden Katalysatoren bei −30 bis 90°C in einem selbstreinigenden, kühlbaren Schneckenreaktor unter kontinuierlicher Zudosierung des freien Polyisocyanats und des Dimerisierungskatalysators, dadurch gekennzeichnet, daß man die Umsetzung in Gegenwart von 3 bis 90 Gew.-%, bezogen auf Gesamtgemisch, eines Hydroxylgruppen aufweisenden Zusatzmittels durchführt, welches entweder (i) aus einer mindestens eine alkoholisch gebundene Hydroxylgruppe aufweisenden Verbindung des Molekulargewichtsbereichs 62 bis 10 000 oder (ii) aus einem Gemisch von mehreren, mindestens eine alkoholisch gebundene Hydroxylgruppe aufweisenden Verbindungen des mittleren Molekulargewichts 62 bis 10 000 besteht, und wobei man das Hilfsmittel dem Reaktionsgemisch spätestens unmittelbar vor der durch die Dimerisierungsreaktion bewirkten Verfestigung des Ausgangsisocyanats hinzufügt.

Gegenstand der vorliegenden Erfindung ist auch die Verwendung der nach diesem Verfahren erhältlichen Verfahrensprodukte als Aufbaukomponente bei der Herstellung von Polyurethankunststoffen nach dem Isocyanat-Polyadditionsverfahren.

Für die Durchführung des erfindungsgemäßen Verfahrens sind Schneckenmaschinen, wie sie dem Stand der Technik entsprechen und z. B. von H. Herrmann in »Schneckenmaschinen in der Verfahrenstechnik«, Springer-Verlag, Berlin-Heidelberg-New York, 1972, S. 161−170 beschrieben wurden, geeignet. Besonders geeignet sind mehrwellige, bevorzugt zweiwellige zwei- und dreigängige Schneckenmaschinen mit gleichsinnig rotierenden, kämmenden Schneckenwellen, die sich ständig gegenseitig und die innere Gehäusewand abschaben, beispielsweise gemäß DE-PS 862 668.

Vorteilhaft werden die Schneckenwellen neben gewindeförmig ausgebildeten »Förderelementen«, beispielsweise in Förderrichtung gesehen kurz nach der Einspeisestelle des Katalysators mit speziellen »Knetelementen« wie sie z. B. in der DE-PS 813 154 und DE-PS 940 109 beschrieben sind, die eine erhöhte Scher- und Mischwirkung haben, bestückt. Auch an anderen Stellen in der Schneckenmaschine, z. B. in der Nähe des Produktaustritts können derartige Knetelemente mit Vorteil eingesetzt werden.

Die Rohstoffe werden bei der Durchführung des erfindungsgemäßen Verfahrens mit geeigneten handelsüblichen Pumpen wie z. B. Kolben-, Membran-, Drehschieber-, Zahnradpumpen, gegebenenfalls vorvermischt, Feststoffe mit Hilfe von geeigneten Feststoffdosiergeräten wie z. B. Schüttelrinnen, Dosierschnecken, Dosierbandwaagen kontiuierlich im gewünschten Verhältnis in den kühl- und gegebenenfalls heizbaren Schneckenreaktor eingebracht.

Obwohl zur Vermeidung der Bildung von Nebenprodukten, wie z. B. Isocyanuratharzen, eine bestimmte verhältnismäßig niedrig liegende Temperatur im Reaktor nicht überschritten werden sollte und eine verhältnismäßig hohe Reaktionswärme abgeführt werden muß, ist es bisweilen vorteilhaft, Rohstoffe in erwärmtem Zustand in den Schneckenreaktor einzuspeisen. Das ist z. B. dann günstig, wenn ein Ausgangsprodukt bei Raumtemperatur nicht in flüssiger Form vorliegt.

Für das erfindungsgemäße Verfahren werden als Ausgangsverbindungen insbesondere aromatische Polyisocyanate der Formel

$R(NCO)_{n'}$,

eingesetzt,
in welcher

n für eine ganze oder (im Falle der Verwendung von Isocyanatgemischen im statistischen Mittel) gebrochene Zahl von 1,5 bis 3, vorzugsweise für 2 steht und

R für einen gegebenenfalls durch einen oder mehrere Alkyl-, Cycloalkyl-, Alkoxy-, Phenoxy- oder Halogenreste substituierten und/oder gegebenenfalls Alkylenreste zwischen aromatischen Ringen als Brückenglieder aufweisenden aromatischen Kohlenwasserstoffrest mit insgesamt 6 bis 30 Kohlenstoffatomen, vorzugsweise für einen gegebenenfalls Methyl-substituierten oder Methylenbrücken aufweisenden aromatischen Kohlenwasserstoffrest mit insgesamt 6 bis 15 Kohlenstoffatomen steht.

Beispiele für derartige aromatische Diisocyanate sind 2,4-Diisocyanatotoluol, 2,6-Diisocyanatotoluol, aus diesen Isomeren bestehende Gemische, gegebenenfalls alkyl- oder halogensubstituiertes 4,4'-Diisocyanatodiphenylmethan und dessen Isomere, 4,4'-Diisocyanatodiphenylpropan, 1,4-Diisocyanato-2-chlor-benzol, 4,4'-Diisocyanato-3,3'-dichlordiphenylmethan, 1,4-Diisocyanato-3-methoxybenzol, 1,4-Diisocyanato-3-phenoxybenzol, Diisocyanato-dimethyl-biphenyl. Bevorzugt bei dem erfindungsgemäßen Verfahren einzusetzende Diisocyanate sind 2,4-Diisocyanatotoluol, 2,6-Diisocyanatotoluol, aus diesen Isomeren bestehende Gemische oder 4,4'-Diisocyanatodiphenylmethan. 2,4-Diisocyanatotoluol ist besonders bevorzugt. In speziellen Fällen können auch monofunktionelle Isocyanate, wie z. B. Phenyl- oder p-Tolylisocyanat, in Mengen bis zu 50 NCO-Äquivalentprozent, bezogen auf Gesamtmenge an Ausgangsisocyanaten oder auch, bevorzugt als Mischkomponenten, tri- und höherfunktionelle aromatische Polyisocyanate verwendet werden, wie beispielsweise die in den Phosgenierungsprodukten von Analin/Formaldehyd-Kondensaten stets vorliegenden höher als difunktionellen Polyisocyanate der Diphenylmethanreihe, so daß beim erfindungsgemäßen Verfahren derartige Phosgenierungsprodukte als Polyisocyanat-Komponente zum Einsatz gelangen, oder auch modifizierte, Urethan- oder Harnstoffgruppen aufweisende Polyisocyanate, beispielsweise das Additionsprodukt aus 5 Mol 4,4'-Diphenylmethandiisocyanat und 1 Mol Tripropylenglykol. Bei Verwendung hochschmelzender Diisocyanate können geringe Mengen organischer Lösungsmittel zur Erniedrigung des Schmelzpunktes dienlich sein. Vorzugsweise werden solche Polyisocyanate eingesetzt, die unter den Temperaturbedingungen des erfindungsgemäßen Verfahrens flüssig sind.

Selbstverständlich können auch Mischungen der genannten Diisocyanate eingesetzt werden.

Bei dem erfindungswesentlichen Zusatzmittel handelt es sich entweder um

i) mindestens eine alkoholisch gebundene Hydroxylgruppe aufweisende organische Verbindungen des Molekulargewichtsbereichs 62 bis 10 000 oder um

ii) Gemische von mindestens eine alkoholisch gebundene Hydroxylgruppe aufweisenden Verbindungen eines mittleren Molekulargewichts von 62 bis 10 000.

Bevorzugte Zusatzmittel (i) sind 2 oder 3 alkoholisch gebundene Hydroxylgruppen aufweisende Verbindungen des Molekulargewichts 400 bis 6000, insbesondere di- oder trifunktionelle aliphatische Polyether- oder Polyesterpolyole dieses Molekulargewichtsbereichs.

Bevorzugte Zusatzmittel (ii) sind Gemische von mindestens eine alkoholische Hydroxylgruppe aufweisenden organischen Verbindungen einer mittleren OH-Funktionalität von 1,5 bis 3 des mittleren Molekulargewichts 400 bis 6000, insbesondere einfache Alkanpolyole des Molekulargewichts 62 bis 400, sowie Polyether- und/oder Polyesterpolyole der zuletzt genannten Art aufweisende Polyolgemische einer mittleren OH-Funktionalität von 2 bis 3 und eines mittleren Molekulargewichts von 400 bis 6000.

Grundsätzlich können als erfindungswesentliches Zusatzmittel alle in der Polyurethanchemie an sich bekannten, alkoholische Hydroxylgruppen aufweisenden Verbindungen wie insbesondere die bereits genannten bevorzugten Polyether- oder Polyesterpolyole, gegebenenfalls im Gemisch mit einfachen Alkanpolyolen, oder auch die allerdings weniger bevorzugten Polythioether-, Polyacetal- oder Polycarbonatpolyole, Hydroxylgruppen aufweisenden Polymerisate wie z. B. Polyhydroxypolyacrylate oder die bekannten Hydroxylgruppen aufweisenden natürlichen Öle eingesetzt werden.

Zu den geeigneten Hydroxylgruppen aufweisenden Verbindungen, die als solche oder als Gemisch

zur Anwendung gelangen, gehören beispielsweise 2- und 3wertige niedermolekulare Alkohole wie Ethylen-, Propylen-, Butylen-, Hexamethylen-, Di-, Tri- oder höhere Polyalkylenglykole, Glycerin oder Trimethylolpropan, sowie die in der Polyurethanchemie an sich bekannten höhermolekularen Polyhydroxylverbindungen, insbesondere Polyesterpolyole bzw. Polyetherpolyole, wie sie beispielsweise in US-PS 4 033 912, Kolonne 5, Zeile 14 bis Kolonne 6, Zeile 12 bzw. in den dort erwähnten Literaturstellen beispielhaft aufgezählt werden. Beispiele der erfindungsgemäß ebenfalls geeigneten, jedoch weniger bevorzugten, oben erwähnten Polyhydroxylverbindungen finden sich in US-PS 4 033 912, Kolonne 6, Zeilen 13—56 bzw. in den dort erwähnten Literaturstellen. Neben diesen Polyhydroxylverbindungen können als Zusatzmittel (i) auch einwertige Alkohole wie z. B. n-Butanol, Isooctanol oder n-Dodecanol eingesetzt werden, obwohl der Einsatz derartiger Hydroxylverbindungen als alleiniges Zusatzmittel gegenüber der Verwendung von höherfunktionellen Polyolen weniger bevorzugt ist. Einwertige Alkohole wie z. B. Methanol, Ethanol oder die zuletzt genannten einwertigen Alkohole können jedoch im Falle der Verwendung eines Alkoholgemischs (ii) als erfindungswesentliches Zusatzmittel in diesem Gemisch vorliegen, soweit die o. g. Bedingungen bezüglich der mittleren OH-Funktionalität und des mittleren Molekulargewichts eingehalten werden.

Die erfindungswesentlichen Zusatzmittel können neben den alkoholischen Hydroxylgruppen auch noch andere gegenüber Isocyanatgruppen reaktionsfähige Gruppen wie insbesondere Urethan-, Carboxyl- oder Sulfhydrylgruppen aufweisen, vorausgesetzt, daß diese zusätzlichen NCO-reaktiven Gruppen gegenüber Isocyanatgruppen keine höhere Reaktivität aufweisen als die alkoholischen Hydroxylgruppen. Vorzugsweise werden jedoch solche Zusatzmittel (i) bzw. (ii) eingesetzt, die als NCO-reaktive Gruppen ausschließlich oder praktisch ausschließlich alkoholische Hydroxylgruppen aufweisen.

Beim erfindungsgemäßen Verfahren werden insbesondere solche Hydroxylgruppen aufweisenden Verbindungen eingesetzt, die unter den Temperaturbedingungen des erfindungsgemäßen Verfahrens, insbesondere unterhalb 80° C und besonders bevorzugt unterhalb 55° C, flüssig sind.

Die als erfindungswesentliche Zusatzmittel zum Einsatz gelangenden, alkoholische Hydroxylgruppen aufweisenden Verbindungen werden in Mengen von 3 bis 90 Gew.-%, vorzugsweise 5 bis 70 Gew.-% und insbesondere 10 bis 60 Gew.-%, bezogen auf Gesamtgemisch, eingesetzt.

Für das erfindungsgemäße Verfahren geeignete Katalysatoren sind alle beliebigen, die Dimerisierung von aromatischen Polyisocyanaten unter Bildung von Uretdiongruppen beschleunigenden Substanzen. Bevorzugt werden tertiäre aliphatische bzw. heterocyclische Amine, wie z. B. Triethylamin, Tri-n-propylamin, N-Methyl- und N-Ethylmorpholin und Pyridin eingesetzt. Besonders bevorzugt sind jedoch Phosphine der Formel

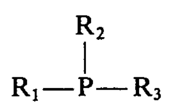

in welcher

$R_1$ einen aliphatischen Kohlenwasserstoffrest mit 1 bis 18 Kohlenstoffatomen bedeutet und
$R_2$ sowie $R_3$ für gleiche oder verschiedene Reste stehen und einen aliphatischen Kohlenwasserstoffrest mit 1 bis 18 Kohlenstoffatomen oder einen Phenylrest bedeuten.

Beim erfindungsgemäßen Verfahren werden die Katalysatoren in solchen Mengen eingesetzt, daß die Dimerisierungsreaktion bei der gewählten nachstehend näher definierten Reaktionstemperatur praktisch quantitativ innerhalb von 60 Minuten, bevorzugt innerhalb 1 bis 3 Minuten, erfolgt. Im allgemeinen werden die Katalysatoren in Mengen von 0,001 bis 3 Gew.-%, vorzugsweise 0,05 bis 2,5 Gew.-%, bezogen auf zu dimerisierendes Ausgangsdiisocyanat eingesetzt. Zur exakten Dosierung geringerer Katalysatormengen ist es vorteilhaft, den Katalysator in einem organischen Lösungsmittel zu lösen. Dabei sollte jedoch die Menge des verwendeten Lösungsmittels, 20 Gew.-%, bevorzugt 5 Gew.-%, bezogen auf das Isocyanat, nicht überschreiten.

Das erfindungsgemäße Verfahren wird vorzugsweise lösungsmittelfrei durchgeführt. Die obengenannte geringe, gegebenenfalls zur Auflösung des Katalysators verwendete Lösungsmittelmenge stört jedoch nicht. Diese geringen Mengen Lösungsmittel können in einfacher Weise während oder nach der Reaktion in der Schneckenmaschine ausgedampft werden. Bei extrem großem Durchsatz und besonders intensiver Kühlung können natürlich auch höhere Katalysatormengen verwendet werden. Man wird natürlich möglichst niedrige Katalysatormengen anstreben, um ein Produkt von hohem Reinheitsgrad zu erhalten.

Beim erfindungsgemäßen Verfahren ist eine Einhaltung eines Temperaturbereichs von −30 bis +90° C wesentlich. Dies kann auf einfache Weise dadurch erreicht werden, daß der Reaktor mit mehreren unabhängig voneinander regulierbaren Temperaturzonen ausgestattet wird. Dabei muß natürlich dort am stärksten gekühlt werden, wo die größte Wärmemenge freigesetzt wird. Andererseits darf die Temperatur auch nicht zu tief gehalten werden, da dann die Reaktionsgeschwindigkeit zu niedrig wird. Gegebenenfalls kann der Reaktor am Einfüllstutzen sogar erwärmt werden,

dann nämlich, wenn festes Ausgangsprodukt vor der Reaktion aufgeschmolzen werden soll.

Die Temperatur des Reaktionsproduktes darf während der Uretdionbildung und vor Vernichtung des Katalysators 90°C nicht überschreiten. Bevorzugt sind Reaktionstemperaturen von −20 bis 70°C, besonders bevorzugt 0 bis 60°C.

Im allgemeinen wird zum Abbruch der Reaktion der Katalysator mit Katalysatorgiften, wie z. B. Alkylierungsmitteln, elementarem Schwefel, Luftsauerstoff oder Sauerstoff abgebenden Verbindungen vernichtet. Das kann z. B. in einem Mischer, einem Kneter, Extruder usw. geschehen. In einer bevorzugten Ausführungsform wird der Katalysator bereits am Ende des Schneckenreaktors vernichtet, in dem die Uretdionbildung durchgeführt wird.

Für viele Anwendungen der nach dem erfindungsgemäßen Verfahren herzustellenden Produkte stört es jedoch nicht, wenn der Katalysator erhalten bleibt.

Bei der Durchführung des erfindungsgemäßen Verfahrens werden das Ausgangspolyisocyanat und die als erfindungswesentliche Zusatzmittel dienenden Hydroxylgruppen aufweisenden Verbindungen entweder getrennt oder bereits (z. B. in einem Durchflußmischer) vorgemischt in die Schneckenmaschine eindosiert. Der Katalysator wird vorzugsweise in die bereits homogene Mischung aus Polyisocyanat und Hydroxylverbindung dosiert, kann aber auch im Zusatzmittel gelöst oder gleichmäßig verteilt sein. Grundsätzlich ist es auch möglich, das Zusatzmittel oder einen Teil davon dem Reaktionsgemisch hinter dem Schneckeneingang an einer beliebigen Stelle vor der Verfestigung des Ausgangsisocyanats zuzudosieren.

Die Produkttemperaturen im Schneckenreaktor liegen erfindungsgemäß zwischen −30 und +90°C, die Drehzahlen der Schneckenwellen in der Regel zwischen 20 und 300 $min^{-1}$.

In einer besonderen Ausführungsform des erfindungsgemäßen Verfahrens wird die Reaktion z. B. über die Schneckendrehzahl, den Durchsatz und/oder die Reaktionstemperatur so gesteuert, daß der Katalysator bereits nach unvollständiger Umsetzung, vorzugsweise innerhalb des Schneckenreaktors, vernichtet wird. Dabei kann die Reaktion so gelenkt werden, daß man einen Umsetzungsgrad von beispielsweise 30 oder 70%, bevorzugt mindestens 50%, erhält. Diese Ausführungsform bietet die Vorteile, daß die Zahl der Nebenreaktionen besonders klein ist und eine verhältnismäßig niedrigviskose, leicht zu handhabende Paste entsteht.

In einer anderen speziellen Ausführungsform wird während oder nach der Katalysatorvernichtung ein weiteres, bevorzugt flüssiges Polyisocyanat, insbesondere der oben beispielhaft genannten Art, bevorzugt kontinuierlich zudosiert und gegebenenfalls, bevorzugt unterhalb 80°C, mit den Hydroxylgruppen des Zusatzmittels zur Reaktion gebracht.

Es ist überraschend, daß nach dem erfindungsgemäßen Verfahren hergestelltes Uretdionpolyisocyanat in Form von sehr feinteiligen Pasten oder Pulvern in dem erfindungsgemäß zu verwendenden Schneckenreaktor anfällt, wobei das Hydroxylgruppen enthaltende Zusatzmittel praktisch nur als Dispergiermittel wirkt. Die Reaktion zwischen Isocyanat- und Hydroxylgruppen, die erwartungsgemäß zu mit beträchtlichen Mengen Bis- und Oligourethan verunreinigten Uretdionpolyisocyanaten hätte führen können, hätte einen Einfluß auf den Erweichungsbereich des Dimerisierungsproduktes gehabt. Diese naheliegende Polyadditionsreaktion findet jedoch nicht statt oder ist in Konkurrenz zur Dimerisatbildung vernachlässigbar gering. Vielmehr fallen die Uretdionpolyisocyanate nach dem erfindungsgemäßen Verfahren nicht wie bei der Dimerisierung in Substanz in Form mehr oder weniger großer Klumpen an, sondern so feindispers, daß sie für die Weiterverarbeitung nicht mehr gemahlen zu werden brauchen.

Um einen wirtschaftlichen Durchsatz zu erreichen, ist es durchaus möglich, die Reaktion in einem der erfindungsgemäß zu verwendenden Mehrwellenschneckenmaschine nachzuschaltenden, vorzugsweise kontinuierlich arbeitenden zweiten Reaktor gleicher oder anderer Art zu Ende zu führen und/oder darin den Dimerisierungskatalysator zu vernichten und/oder das Reaktionsprodukt mit einem weiteren unter den angewandten Bedingungen mit dem Uretdiongruppen aufweisenden Polyisocyanat nicht reagierenden, vorzugsweise flüssigen Stoff zu mischen.

Gemäß einer speziellen Ausführungsform des erfindungsgemäßen Verfahrens können den Verfahrensprodukten während oder nach der Vernichtung des Katalysators auch noch weitere Hydroxylgruppen aufweisende Verbindungen der bereits oben beispielhaft genannten Art zugemischt werden, um beispielsweise das Äquivalentverhältnis zwischen Isocyanat- und Uretdiongruppen einerseits und Hydroxylgruppen andererseits der beabsichtigten Endverwendung anzupassen, so daß beispielsweise unmittelbar als Endprodukte Systeme erhalten werden, die durch Hitzeeinwirkung in hochmolekulare Polyurethankunststoffe überführbar sind. Derartige Systeme liegen auch ohne nachträgliche Zugabe einer zweiten Polyisocyanatkomponente vor, wenn das Äquivalentverhältnis zwischen Isocyanatgruppen des Ausgangspolyisocyanats und Hydroxylgruppen der Zusatzmittelkomponente um 1 : 1 liegt. Im allgemeinen werden jedoch bei der Durchführung des erfindungsgemäßen Verfahrens die Mengenverhältnisse der einzelnen Komponenten so bemessen, daß ein Überschuß von Isocyanat- und Uretdiongruppen gegenüber Hydroxylgruppen vorliegt. Dieser Überschuß kann dann, wie dargelegt, durch Zugabe von weiterem Polyisocyanat im Anschluß an die erfindungsgemäße Dimerisierungsreaktion weiter erhöht werden. Derartige erfindungsgemäße Systeme, in denen ein Überschuß an Isocyanat- und Uretdiongruppen gegenüber Hydroxylgruppen vorliegt, eignen sich als Polyisocyanat-Komponente bei der Herstellung von Polyurethankunststoffen nach dem Isocyanat-Polyadditionsverfahren, wobei diese Systeme sowohl freie Isocyanatgruppen, d. h. bereits bei Raumtemperatur oder mäßig erhöhter Temperatur mit einer Polyhydroxylkomponente reaktionsfähige Zentren und Uretdiongruppen aufweisen, die bekanntlich mit Hydroxylgruppen erst bei Einwirkung höherer Temperaturen abreagieren. Diese erfindungsgemäßen Verfahrensprodukte eignen sich somit insbesondere zur Herstellung von Polyurethankunststoffen nach einem Zweistufenverfahren, bei welchem bei Raumtemperatur oder mäßig erhöhter Temperatur zunächst mit einer Polyhydroxylkomponente ein noch verformbares Vorprodukt erhalten wird, welches verformbar ist und durch Hitzeeinwirkung in den vernetzten Endzustand überführt werden kann. Im Falle der Verwendung von nur geringen Mengen an erfindungswesentlichem Zusatzmittel entstehen beim erfindungsgemäßen Verfahren pulverförmige Verfahrensprodukte, die insbesondere in Kombination mit festen, pulverisierbaren Polyhydroxylverbindungen sehr gut zu Pulverlacken verarbeitbar sind. Zur Herstellung derartiger Pulverlacke empfiehlt sich beispielsweise der Einsatz von geringen Mengen erfindungswesentlicher Zusätze (i) oder (ii) innerhalb der o. g. Grenzen, sowie der nachträgliche Zusatz von bei Raumtemperatur festen Polyhydroxylverbindungen, beispielsweise von bei Raumtemperatur festen Polyesterpolyolen.

Die erfindungsgemäßen Verfahrensprodukte können auch durch Zusatz weiterer Hilfsmittel, wie beispielsweise Lösungsmitteln, Plastifizierungsmitteln, Farbstoffen, Emulgatoren, anorganischen oder organischen Füllstoffen, Verlaufmitteln, Stabilisatoren, Antioxidantien usw. modifiziert werden. Die Zugabe dieser Zusatzstoffe kann beispielsweise am Schneckenanfang oder -ende erfolgen.

Die Verfahrensprodukte können auch zur Verbesserung der Haftung von Kunststoffen, einschließlich Gummi an Cord, verwendet werden.

## Beispiel 1

In den Trichter eines Labordoppelwellenschneckenreaktors mit einem äußeren Wellendurchmesser von 32 mm und einer Länge von ca. 1216 mm werden kontinuierlich im Gewichtsverhältnis 8 : 2 auf ca. 40°C erwärmtes 2,4-Toluylendiisocyanat und ein difunktionelles Polyetherpolyol, welches durch Anlagerung von Propylenoxid und Ethylenoxid an 1,2-Propandiol erhalten worden ist, und welches endständig überwiegend primäre Hydroxylgruppen und ein Durchschnittsmolekulargewicht von ca. 4000 aufweist, dosiert. In das 2. von 5 Gehäusen werden kontinuierlich 1,3% Tributylphosphin berechnet auf Toluylendiisocyanat gegeben. Die Versuchsdaten sind aus Tabelle 1 zu entnehmen. In einer kurzen Compoundierschneckenmaschine werden je Gewichtsteil Produkt 0,003 Gewichtsteile Schwefel in die hochviskose Paste eingeknetet. Die Feststoffteilchen haben eine Korngröße von unter 0,02 mm.

Tabelle 1

| | Beispiel | | |
|---|---|---|---|
| | 1 A | 1 B | 1 C |
| Gesamtdurchsatz (kg/h) | 12 | 15 | 24 |
| Wellendrehzahl ($min^{-1}$) | 150 | 175 | 275 |
| Verweilzeit ca. (min) | 2,1 | 1,6 | 1,1 |
| Gehäusetemperaturen (°C) | | | |
| Gehäuse 2 | 23 | 25 | 24 |
| Gehäuse 3 | 12 | 12 | 9 |
| Gehäuse 4 | 16 | 17 | 16 |
| Gehäuse 5 | −14 | −14 | −14 |
| Produktaustrittstemperatur (°C) | 30 | 35 | 44 |
| Ausbeute (%)[1] | 88 | 89 | 99 |

[1]) Ausbeute an Uretdiondiisocyanat berechnet auf eingesetztes Toluylendiisocyanat.

Beispiel 2

In den Einspeisetrichter des im Beispiel beschriebenen Schneckenreaktors werden kontinuierlich auf ca. 40° C erwärmtes 2,4-Toluylendiisocyanat und der im Beispiel 1 beschriebene Mischpolyether im Gewichtsverhältnis 95 : 5 dosiert. In das zweite Gehäuse werden 1,3 Gew.-% Tributylphosphin (ber. auf Diisocyanat) dosiert. Die Teilchengröße des entstehenden Uretdiondiisocyanats liegt unter 0,03 mm. Die Versuchsbedingungen sind in Tabelle 2 zusammengestellt.

Tabelle 2

| | Beispiel | | |
|---|---|---|---|
| | 2 A | 2 B | 2 C |
| Gesamtdurchsatz (kg/h) | 12 | 18 | 24 |
| Wellendrehzahl ($min^{-1}$) | 130 | 190 | 225 |
| Verweilzeit ca. (min) | 2,1 | 1,4 | 1,0 |
| Gehäusetemperaturen (°C) | −16 bis +12 | −16 bis +20 | −15 bis +12 |
| Produktaustrittstemperatur (°C) | 20 | 49 | 55 |
| Ausbeute (%)[1] | 97 | 96 | 96 |

Beispiel 3

Es wird wie in Beispiel 2 beschrieben verfahren, jedoch werden Toluylendiisocyanat und Polyether im Gewichtsverhältnis 1 : 1 eingesetzt. Es wird eine stark klebrige Paste erhalten, in der die Uretdiondiisocyanatteilchen einen Durchmesser unter 0,02 mm haben.

Beispiel 4

In den Dosiertrichter des Schneckenreaktors gemäß Beispiel 1 wird die in einem kurzen statischen Mischer erhaltene, auf 40° C erwärmte Mischung aus 8 Gew.-Teilen 2,4-Toluylendiisocyanat und 3,6 Gew.-Teilen des im Beispiel 1 beschriebenen Polyethers, in das zweite Reaktorgehäuse 1,3 Gew.-% (ber. auf Toluylendiisocyanat) Tributylphosphin und in die Mitte der Schneckenmaschine die gut gerührte Suspension von 0,048 Gew.-Teilen Schwefel in 0,4 Teilen des gleichen Polyethers eingespeist. Zu Beginn des letzten Viertels des Reaktors werden außerdem 5 Gew.-Teile eines rohen, bei Raumtemperatur flüssigen 4,4'-Diisocyanatodiphenylmethans kontinuierlich zudosiert. Es wird eine freifließende Paste erhalten. Reaktionsbedingungen siehe Tabelle 3.

Tabelle 3

| | Beispiel | |
|---|---|---|
| | 4 A | 4 B |
| Gesamtdurchsatz (kg/h) | 17 | 17 |
| Wellendrehzahl ($min^{-1}$) | 300 | 175 |
| Gehäusetemperaturen (°C) | 39—52 | 41—53 |
| Produktaustrittstemperatur (°C) | 48 | 49 |
| Ausbeute (%)[1] | 68 | 82 |
| Feststoffteilchengröße (μm) ca. | 2 × 10 | 3 × 15 |

**Patentansprüche**

1. Verfahren zur kontinuierlichen Herstellung von Uretdiongruppen aufweisenden Polyisocyanaten durch Dimerisierung von uretdiongruppenfreien aromatischen Polyisocyanaten in Gegenwart von die Dimerisierung von Isocyanaten beschleunigenden Katalysatoren bei −30 bis 90°C in einem selbstreinigenden, kühlbaren Schneckenreaktor unter kontinuierlicher Zudosierung des freien Polyisocyanats und des Dimerisierungskatalysators, dadurch gekennzeichnet, daß man die Umsetzung in Gegenwart von 3 bis 90 Gew.-%, bezogen auf Gesamtgemisch, eines Hydroxylgruppen aufweisenden Zusatzmittels durchführt, welches entweder (i) aus einer mindestens eine alkoholisch gebundene Hydroxylgruppe aufweisenden Verbindung des Molekulargewichtsbereichs 62 bis 10 000 oder (ii) aus einem Gemisch von mehreren, mindestens eine alkoholisch gebundene Hydroxylgruppe aufweisenden Verbindungen des mittleren Molekulargewichts 62 bis 10 000 besteht, und wobei man das Hilfsmittel dem Reaktionsgemisch spätestens unmittelbar vor der durch die Dimerisierungsreaktion bewirkten Verfestigung des Ausgangsisocyanats hinzufügt.

2. Verfahren gemäß Anspruch 1, dadurch gekennzeichnet, daß man als Hydroxylgruppen aufweisendes Hilfsmittel (i) eine Polyhydroxylverbindung mit 2 oder 3 alkoholisch gebundenen Hydroxylgruppen und einem Molekulargewicht von 400 bis 6000 oder (ii) ein Gemisch von alkoholisch gebundene Hydroxylgruppen aufweisenden Verbindungen einer mittleren OH-Funktionalität von 1,5 bis 3 und einem mittleren Molekulargewicht von 400 bis 6000 verwendet.

3. Verfahren gemäß Anspruch 1 und 2, dadurch gekennzeichnet, daß man die Dimerisierungsreaktion durch Vermischen des Reaktionsgemischs mit einem Katalysatorengift für den Dimerisierungskatalysator abbricht.

4. Verfahren gemäß Anspruch 1 bis 3, dadurch gekennzeichnet, daß man das Reaktionsgemisch während oder nach der Katalysatorvernichtung mit weiteren Polyisocyanaten oder alkoholische Hydroxylgruppen aufweisenden Verbindungen vermischt.

5. Verwendung der gemäß Anspruch 1 bis 4 erhaltenen Uretdiongruppen aufweisenden Polyisocyanate als Aufbaukomponente bei der Herstellung von Polyurethankunststoffen nach dem Isocyanat-Polyadditionsverfahren.

**Claims**

1. Process for the continuous preparation of polyisocyanates containing uretdione groups by the dimerisation of aromatic polyisocyanates which are free from uretdione groups in the presence of catalysts which accelerate the dimerisation of isocyanates, which preparation is carried out at −30 to 90°C in a self-cleaning, coolable screw reactor with continuous feeding in of the free polyisocyanate and of the dimerisation catalyst, characterised in that the reaction is carried out in the presence of 3 to 90% by weight, based on the whole mixture, of an additive containing hydroxyl groups, which additive consists of either (i) a compound within the molecular weight range of from 62 to 10,000 containing at least one alcoholically bound hydroxyl group or (ii) a mixture of several compounds having an average molecular weight within the range of from 62 to 10,000 containing at least one alcoholically bound hydroxyl group, the auxiliary agent being added to the reaction mixture not later than immediately before the isocyanate used as starting material solidifies due to the dimerisation reaction.

2. Process according to claim 1, characterised in that the hydroxyl group-containing auxiliary agent (i) is a polyhydroxyl compound containing 2 or 3 alcoholically bound hydroxyl groups and having a molecular weight of from 400 to 6000 or (ii) a mixture of compounds containing alcoholically bound hydroxyl groups and having an average hydroxyl functionality of from 1.5 to 3 and an average molecular weight in the range of from 400 to 6000.

3. Process according to claims 1 and 2, characterised in that the dimerisation reaction is stopped by mixing the reaction mixture with a catalyst poison for the dimerisation catalyst.

4. Process according to claims 1 to 3, characterised in that the reaction mixture is mixed with additional polyisocyanates or compounds containing alcoholic hydroxyl groups during or after destruction of the catalyst.

5. Use of the uretdione group-containing polyisocyanates obtained according to claims 1 to 4 as starting components for the production of polyurethane plastics by the isocyanate polyaddition process.

**Revendications**

1. Procédé de production continue de polyisocyanates porteurs de groupes uret-dione par dimérisation de polyisocyanates aromatiques dépourvus de groupes uret-dione en présence de catalyseurs accélérant la dimérisation d'isocyanates à des températures de −30 à 90°C dans un réacteur à vis sans fin autonettoyant, pouvant être refroidi, avec addition continue du polyisocyanate libre et du catalyseur de dimérisation, caractérisé en ce qu'on conduit la réaction en présence de 3 à

90% en poids, par rapport au mélange total, d'un additif porteur de groupes hydroxyle qui est formé (i) ou bien d'un composé portant au moins un groupe hydroxyle en liaison alcoolique, d'un poids moléculaire compris dans la plage de 62 à 10 000, ou bien (ii) d'un mélange de plusieurs composés portant au moins un groupe hydroxyle en liaison alcoolique, d'un poids moléculaire moyen de 62 à 10 000, et on ajoute alors l'additif au mélange réactionnel au plus tard juste avant la solidification de l'isocyanate de départ, provoquée par la réaction de dimérisation.

2. Procédé suivant la revendication 1, caractérisé en ce qu'on utilise comme additif porteur de groupes hydroxyle (i) un composé polyhydroxylique présentant 2 ou 3 groupes hydroxyle en liaison alcoolique et ayant un poids moléculaire de 400 à 6000 ou bien (ii) un mélange de composés porteurs de groupes hydroxyle en liaison alcoolique, ayant une fonctionnalité OH moyenne de 1,5 à 3 et un poids moléculaire moyen de 400 à 6000.

3. Procédé suivant les revendications 1 et 2, caractérisé en ce qu'on arrête la réaction de dimérisation par adjonction au mélange réactionnel d'un poison pour le catalyseur de dimérisation.

4. Procédé suivant les revendications 1 à 3, caractérisé en ce qu'on adjoint au mélange réactionnel pendant ou après la désactivation du catalyseur, d'autres polyisocyanates ou d'autres composés porteurs de groupes hydroxyle alcooliques.

5. Utilisation des polyisocyanates porteurs de groupes uret-dione obtenus suivant les revendications 1 à 4 comme composant structural dans la production de matières plastiques du type uréthanne par le procédé de polyaddition d'un isocyanate.